# EUROPEAN PATENT APPLICATION

(11) **EP 1 760 159 A1**
(43) Date of publication of application: **07.03.2007**
(21) Application number: 05018950.5
(22) Date of filing: 31.08.2005
(51) Int. Cl.: C12Q 1/68

(54) **A method of electrophoresis of nucleic acid molecules**

(71) Applicant: Max-Planck-Gesellschaft zur Förderung der Wissenschaften e.V., Berlin (DE)
(72) Inventor: Soldatov, Aleksey, 14163 Berlin (DE); Lehrach, Hans, 14129 Berlin (DE); Borodina, Tatiana, 14163 Berlin (DE)
(74) Representative: Vossius & Partner

(57) **Abstract**

The present invention relates to a highly parallel method for performing an electrophoresis comprising the steps of (a) loading a sample containing duplex nucleic acid molecules onto a porous matrix, wherein one strand of each duplex nucleic acid molecule is attached to a macromolecular structure or solid material; (b) heating the sample of step (a), thereby melting said duplex nucleic acid molecules; and, concomitantly or subsequently, (c) performing electrophoresis.

The present invention furthermore relates to a method of sequencing which makes use of the method of the present invention.

SEQUENCE LISTING

<110> Max-Planck-Gesellschaft zur Förderung der Wissenschaften
e.V.

<120> A method of electrophoresis of nucleic acid molecules

<130> L 1293 EP

<140> 05 01 8950.5
<141> 2005-08-31

<160> 16

<170> PatentIn version 3.3

<210> 1
<211> 29
<212> DNA
<213> Artificial

<220>
<223> oligonucleotide, complementary to pBluescript II SK(+) vector DNA

<400> 1
ggaaacagct atgaccatga ttacgccaa 29

<210> 2
<211> 30
<212> DNA
<213> Artificial

<220>
<223> oligonucleotide, complementary to pBluescript II SK(+) vector DNA

<400> 2
ataacaattt cacacaggaa acagctatga 30

<210> 3
<211> 25
<212> DNA
<213> Artificial

<220>
<223> oligonucleotide, complementary to pBluescript II SK(+) vector DNA

<400> 3
gcgcgcaatt aaccctcact aaagg 25

<210> 4
<211> 25
<212> DNA
<213> Artificial

<220>
<223> oligonucleotide, complementary to pBluescript II SK(+) vector DNA

<400> 4
ccctatagtg agtcgtatta cgcgc 25

<210>  5
<211>  22
<212>  DNA
<213>  Artificial

<220>
<223>  synthesized oligonucleotide

<400>  5
ggagcacgct atcccgttag ac                                              22


<210>  6
<211>  54
<212>  DNA
<213>  Artificial

<220>
<223>  synthesized oligonucleotide

<400>  6
cataacagtt tcaattatat attgtgtggg ttaaatgtct aacgggatag cgtg          54


<210>  7
<211>  20
<212>  DNA
<213>  Artificial

<220>
<223>  oligonucleotide, complementary to #template

<400>  7
gcacaattca cgactcacga                                                 20


<210>  8
<211>  30
<212>  DNA
<213>  Artificial

<220>
<223>  oligonucleotide, complementary to #template


<220>
<221>  variation
<222>  (1)..(1)
<223>  5' acrydite modification

<400>  8
gagcatgaat atgtatttgc aactctttag                                     30


<210>  9
<211>  22
<212>  DNA
<213>  Artificial

<220>
<223>  synthesized oligonucleotide


<220>

<221>  variation
<222>  (1)..(1)
<223>  5' acrydite modification

<400>  9
cccgttagac cgatgctgaa ag                                              22


<210>  10
<211>  23
<212>  DNA
<213>  Artificial

<220>
<223>  oligonucleotide, complementary to pBluescript II SK(+) vector DNA


<220>
<221>  variation
<222>  (1)..(1)
<223>  5' acrydite modification

<400>  10
gacgttgtaa aacgacggcc agt                                             23


<210>  11
<211>  29
<212>  DNA
<213>  Artificial

<220>
<223>  synthesized oligonucleotide


<220>
<221>  variation
<222>  (1)..(1)
<223>  5' acrydite modification

<400>  11
ccgacgtttt acaacagttt ggtcttaaa                                       29


<210>  12
<211>  124
<212>  DNA
<213>  Artificial

<220>
<223>  artificial sequence obtained in the procedure described in
       Borodina, T.A., Lehrach, H. and Soldatov, A.V. (2004) Ligation
       detection reaction - TaqMan procedure for single nucleotide
       polymorphism detection on genomic DNA. Ana.l Biochem., 333,


<400>  12
tgcacaattc acgactcacg atccacacgg tctcgcactg gcacgggcac cgacgcttac     60

agttatcctc taaagagttg caaatacata ttcatgctcg gtctaacggg atagcgtggt    120

ggta                                                                 124

<210> 13
<211> 52
<212> DNA
<213> Artificial

<220>
<223> amplified fragment of pBluescript II SK(+) vector DNA,nucleotides
594-645, 52nt

<400> 13
gacgttgtaa aacgacggcc agtgagcgcg cgtaatacga ctcactatag gg 52


<210> 14
<211> 204
<212> DNA
<213> Artificial

<220>
<223> amplified fragment of pBluescript II SK(+) vector DNA,nucleotides
594-797, 204nt

<400> 14
gacgttgtaa aacgacggcc agtgagcgcg cgtaatacga ctcactatag ggcgaattgg 60

gtaccgggcc ccccctcgag gtcgacggta tcgataagct tgatatcgaa ttcctgcagc 120

ccgggggatc cactagttct agagcggccg ccaccgcggt ggagctccag cttttgttcc 180

ctttagtgag ggttaattgc gcgc 204


<210> 15
<211> 233
<212> DNA
<213> Artificial

<220>
<223> amplified fragment of pBluescript II SK(+) vector DNA,nucleotides
594-826, 233nt

<400> 15
gacgttgtaa aacgacggcc agtgagcgcg cgtaatacga ctcactatag ggcgaattgg 60

gtaccgggcc ccccctcgag gtcgacggta tcgataagct tgatatcgaa ttcctgcagc 120

ccgggggatc cactagttct agagcggccg ccaccgcggt ggagctccag cttttgttcc 180

ctttagtgag ggttaattgc gcgcttggcg taatcatggt catagctgtt tcc 233


<210> 16
<211> 249
<212> DNA
<213> Artificial

<220>
<223> amplified fragment of pBluescript II SK(+) vector DNA,nucleotides
594-842, 249nt

<400> 16
gacgttgtaa aacgacggcc agtgagcgcg cgtaatacga ctcactatag ggcgaattgg 60

gtaccgggcc ccccctcgag gtcgacggta tcgataagct tgatatcgaa ttcctgcagc 120

ccgggggatc cactagttct agagcggccg ccaccgcggt ggagctccag cttttgttcc 180

ctttagtgag ggttaattgc gcgcttggcg taatcatggt catagctgtt tcctgtgtga 240

aattgttat 249

## Description

The present invention relates to a method for performing an electrophoresis comprising the steps of (a) loading a sample containing duplex nucleic acid molecules onto a porous matrix, wherein one strand of each duplex nucleic acid molecule is attached to a macromolecular structure or solid material; (b) heating the sample of step (a), thereby melting said duplex nucleic acid molecules; and, concomitantly or subsequently, (c) performing electrophoresis.

The present invention furthermore relates to a method of sequencing which makes use of the method of the present invention.

In this specification, a number of documents are cited. The disclosure content of these documents including manufacturers' manuals is herewith incorporated by reference in its entirety.

Electrophoresis-based sequencing remains the most efficient sequencing procedure in last three decades. Efforts in the refinement of the Sanger sequencing procedure resulted in exponential increase of the cost-effectiveness of the sequencing (bp/$) in these years with doubling time of about 18 months [Shendure et al., 2004]. Nowadays 384-capillary sequencing machine can generate up to 3x10⁶ raw nucleotides per day.

The further increase in the cost-effectiveness is required for many scientific and industrial applications, such as preventative, individual and forensic medicine, cancer research, gene-expression profiling, quantification of splice variants, etc.

Currently, electrophoretic separation is the last stage of the complex multistep procedure which comprises the following steps:
- cloning;
- colonies picking;
- amplification and purification of the PCR products;
- Sanger primer extension;
- gel loading and fragment separation.

The progress in the sequencing technology was related with:
(i) improvement of individual stages of the process (for example: substitution of PCR-amplification by BRCA-amplification; substitution of thick electrophoresis gels by thin gels and microcapillaries; substitution of conventional Sanger sequencing by cycle-sequencing);
(ii) automation (for example: liquid-handling robots; colony-picking machines);
(iii) parallelization of procedure (for example: multichannel pipettes, multiwell plates, multicapillar sequencing machines, etc.).

Despite the above cited progresses, the level of parallelization has reached its limit. Increasing number of channels/wells/capillars would give only a limited improvement in effectiveness.

Further progress in parallelization might be achieved by performing sequencing process on a solid phase. Unlike reactions in liquid phase a great number of individual solid-phase reactions may be performed simultaneously. Examples of parallel solid-phase assays known in the prior art are: gene-expression [Burgess, 2001] and SNP-detection chips [Dong et. al., 2001]; Affymetrix platform for "sequencing by hybridization" [Hacia, 1999]; polonies sequencing reaction [Shendure et.al., 2005], etc. In particular, the gel loading step is still carried out in liquid phase. The prior art, however, did not give any hint or suggestion that the loading step might be adapted nor was their any teaching that adapting the loading step might help increase the efficiency of the method.

As discussed, the methods of the prior art manifest disadvantages. In particular, with respect to the improvement of the effectiveness, the prior art methods had only a reduced potential. Thus, there was a need for methods that overcome said disadvantages. The prior art did not disclose any methods that might be handled easily and overcome the above mentioned disadvantages. The technical problem underlying the present invention was therefore to provide such methods.

The solution to said technical problem is achieved by providing the embodiments characterized in the claims.

Accordingly, the present invention relates to a method for performing an electrophoresis comprising the steps of (a) loading a sample containing duplex nucleic acid molecules onto a porous matrix, wherein one strand of each duplex nucleic acid molecule is attached to a macromolecular structure or solid material; (b) heating the sample of step (a), thereby melting said duplex nucleic acid molecules; and, concomitantly or subsequently, (c) performing electrophoresis.

The term "electrophoresis" as used in connection with the present invention is known for the person skilled in the art. It refers to the separation of charged molecules in an electric field across a porous matrix. In particular, the technique separates molecules by their rate of movement in an electric field. In the case of nucleic acid molecules the molecules are separated by size. [ref.: Sambrook, 2001]

The term "porous matrix" as used in connection with the present invention is known for the person skilled in the art. It refers to the substance in which charged molecules are separated during electrophoresis. This substance is used as a molecular sieve. As the charged molecules migrate through the sieve they become hindered, with large molecules more hindered than smaller ones. The porous matrix restricts convection of liquid and diffusion of separated molecules. The porosity of the matrix can be controlled by the person skilled in the art by selection of material for the matrix and by varying the concentration of this material.

In conventional electrophoresis solid polymers are preferably used as a matrix to provide easy handling and analysis. Most known examples of solid polymers are polyacrylamide and agarose gels.

The solid state of "porous matrix" is not necessary for the capillar electrophoresis, because the capillar itself provides a support for handling. This means that liquid polymers can be used as a molecular sieve. Most known examples of liquid polymers are linear polyacrylamide, polyvinyl alcohol, dextran.

The term "duplex nucleic acid molecule" in connection with the present invention means that said duplex nucleic acid molecule is characterized by two single-stranded nucleic acid molecules which are hybridized to each other thereby forming said duplex nucleic acid molecule. The term may refer both to perfect and imperfect matched duplexes. Preferably, said duplex nucleic acid molecules are characterized by a melting temperature (if measured or calculated for 50nM duplex in 50mM salt solution) higher than 15°C, higher than 20°C and most prefered higher than 30°C.

The term "macromolecular structure" as used in connection with the present invention refers to any molecular structure, which links together all nucleic acid duplexes of one sample. The macromolecular structure may be built up by linear or crosslinked arrangements of building blocks. It may be of organic or inorganic origin. Examples for macromolecular structures are also further described below. In a preferred embodiment of the present invention said macromolecular structure has a molecular mass from 10³ Da to 10⁹ Da, more preferably 10⁴ Da to 10⁸ Da and even more preferred 10⁵ Da to 10⁷ Da.

The term "wherein one strand of the duplex nucleic acid molecule is attached to a macromolecular structure or solid material" in connection with the present invention means that said strand may be attached in a covalent or non covalent manner by procedures known to the person skilled in the art. For example, the covalent attachment of 5' amino- (or phospho-, or thiophosho-, or acrydite-, or thio-) modified nucleic acid molecules to activated polyacrylamide (or polyvinilpirollidone, or dextrane, or dendrimere, etc.) molecules can be carried out by a person skilled in the art [Hermanson, 1996]. Furthermore, non-covalent binding of nucleic acid to macromolecular complex (for example, the binding of nucleic acid molecules through biotin to the complex of crosslinked streptavidine molecules) is also envisaged in connection with the present invention and can be carried out by a person skilled in the art.

The term "solid material" as used in connection with the present invention refers to any solid object which links together all nucleic acid duplexes of one sample. Said object is known to a person skilled in the art and will also be described further below.

The solid material with attached nucleic acids may be prepared by a number of ways known for the person skilled in the art. For example, the covalent attachment of 5' amino- (or phospho-, or thiophosho-, or acrydite-, or thio-) modified nucleic acid molecules to activated polyacrylamide (or silica, of latex, or gold, etc.) particles [Arshady, 1993; Fritzsche and Taton, 2003] may be carried out. Procedures for noncovalent binding of nucleic acid to particles (for example, binding nucleic acid molecules through biotin to streptavidine molecules crosslinked to the solid material [Huang et.al., 1994; Dressman et.al., 2003]) are also known to the person skilled in the art.

As mentioned above and in other terms, the invention resolves the recited technical problem by providing a method for performing electrophoresis. In particular, as mentioned above, in the prior art, the loading of the nucleic acid molecules for electrophoresis was carried out by transferring the samples containing the nucleic acid molecules onto the porous matrix in solution. As explained, this is nowadays achieved by sophisticated liquid-handling equipment. The loading of the samples in solution has however a number of disadvantages:
- loss of significant part of the sample (up to 90-99%) [Paegel, 2003];
- high price of the liquid-handling equipment;
- restricted potential for multiplexation (because it may be achieved only by multiplexation of the number of liquid-handling tools).

In particular, the loss of sample is due to technical problems related with handling of extremely small (less than 1-2µl) volumes of liquid. It is necessary to dilute samples just to supply automatic liquid-handling robot with the required volume of liquid and to prevent drying of the sample because of evaporation. Another problem is related with incomplete transfer of liquid and incomplete loading of nucleic acids into the capillar by electrokinetic injection.

The inventors found, that nucleic acid molecule duplexes attached with one strand to a macromolecular structure or solid material are stable at low temperatures and under denaturing conditions, normally used for sequencing. Furthermore, the inventors found that said nucleic acid molecules are prevented from diffusing into the electrophoretic medium. The inventors could also show that the duplex nucleic acid molecules attached to a macromolecular structure or solid material can be melted by increasing of the temperature just before or during electrophoresis. Thus, by loading the attached nucleic acid molecules onto a porous matrix, it is possible to separate the two strands of the duplex after carrying out the loading procedure. By performing the electrophoresis step, the separated free strands can then migrate through the porous matrix and their size be determined and/or the sequence be assessed.

The above is particular surprising, since it was for the first time possible to load nucleic acid molecules together with a macromolecular structure or solid support to which they are attached directly onto the porous matrix and still be able to carry out electrophoresis. The loading step could thus be carried out in solid phase, avoiding the disadvantageous material consuming loading steps as carried out in the methods of the prior art which are based on a liquid environment, i.e. which are carried out in solution.

The advantages of the above method, as also explained below for the exemplary case of polyacrylamide granules, in particular lies in the possibility to further automatize and parallelize the procedure of electrophoresis, also in view to possible applications in sequencing. The Sanger primer extension reaction may be performed simultaneously for all granules in one tube. Washing of the granules before electrophoresis also may be done for all granules simultaneously. All extended primers will be loaded on the gel, without any loss, because they are associated with granules. These features would make sequencing cheaper, easier and faster.

The single-stranded nucleic acid strands separated from the duplexes then migrate through the porous matrix in electric field. The migration distance may be determined after or during electrophoresis.

It is possible in connection with the method of the present invention to use electrophoretic data (migration distances) to determine the absolute length of said single-stranded nucleic acid strands or compare the length of different nucleic acid strands within the same sample. The absolute length of said single-stranded nucleic acid strands may be determined by comparison of migration distances of said strands with migration distances of reference nucleic acid molecule samples.

The comparison of the length of different nucleic acid molecule strands within the same sample is used in the Sanger sequencing method with four different fluorescent ddNTP-terminators. Electrophoresis data provides relative mobilities of four groups of nucleic acid molecules with 4 different fluorescent markers. This information permits to reconstruct the sequence of the fragment of the nucleic acid molecule of interest.

Experimentally, the inventors could show that when one strand of the duplex nucleic acid molecule is covalently attached to polyacrylamide granules via an acrydite group, said granules may be loaded in a gel at low temperature. The granules are put onto the unpolymerized gel just before polymerization is started. During loading and before electrophoresis starts, covalently bound strands prevent hybridized strands from diffusion into the gel. Immediately before or during electrophoresis duplexes could be melted by heating the whole gel to 70-95°C. Duplexes will then melt and the free strand will move in the electric field.

The advantages of loading samples in granules are: (i) highly parallel sample loading is possible (for example, it is possible to immobilize a thousands of granules in the sequencing gel by loading granules in the gel solution before polymerization); (ii) granules may be very small, i.e. the method of the invention can work for granules with the sizes from 100nm to 3mm, bearing the amount of sample just enough for electrophoresis analysis; (iii) different granules may carry different types of nucleic acids duplexes (since they are immobilized within granules, they would not mix with each other); (iv) enzymatic reactions (for example, sequencing primer extension with dye-terminators) or buffer exchange preceding to electrophoresis may be performed in one tube for many granules simultaneously.

It is also preferred that said duplex nucleic acid molecules which are loaded on a pourous matrix are DNA molecules. In another preferred embodiment of the method of the present invention said nucleic acid molecules are RNA/DNA hybrids, RNA/RNA hybrids or hybrids of DNA or RNA with PNA.

In a preferred embodiment of the method of present invention said electrophoresis is carried out under denaturing conditions.

The term "denaturing conditions" as used in connection with the present invention refers to conditions wherein strand separation of the duplex nucleic acid molecules is achieved.

The skilled person knows various conditions, which result in strand separation. These conditions destroy hydrogen bounds that link together the strands of duplex nucleic acid molecules. Also, by increasing the temperature in the sample, the separation of the strands is enhanced. In particular, the addition to the sample of urea is envisaged, wherein said urea has a preferred final concentration in the sample in the range of 5 to 50% w/v, more preferred in the range of 10% to 45% w/v, and most preferred in the range of 30% to 45% w/v, or of formamide, wherein said formamide has a preferred final concentration in the sample of 5 to 95% w/v. The person skilled in the art also knows that by increasing of pH of the sample, the separation of the strands is enhanced. The high temperature also facilitates strand separation. Preferred temperatures for melting of nucleic acid duplexes is from 30°C to 99°C, preferably in the range of 50°C to 99°C, more preferred in the range of 60°C to 98°C.

In further preferred embodiment of the method of the present invention said loading of the samples on the porous matrix is performed at a temperature wherein the duplex nucleic acid molecules are stable.

In a more preferred embodiment of the method of the present invention said temperature is below 40°C.

Preferably, said temperature is below 30°C, and most preferred below 25°C.

In another preferred embodiment of the method of the present invention said porous matrix is a gel.

In a more preferred embodiment of the method of the present invention said gel is polyacrylamide or agarose gel.

It is preferred in the method of the present invention that a polyacrylamide gel be used which is a standard in electrophoretic separation and provides separation capabilities for a wide range of samples.

In another preferred embodiment of the method of the present invention said porous matrix is a linear polymer. Examples for linear polymers are linear polyacrylamides, polyvinyl alcohols or dextranes.

In a more preferred embodiment of the method of the present invention, said linear polymer is a linear polyacrylamide.

It is preferred in the method of the present invention that a linear polyacrylamide be used, which is a standard in capillary electrophoretic separation for the sequencing.

In another preferred embodiment of the method of the present invention, said macromolecular structure is selected from the group consisting of (a) high molecular weight polymers; (b) concatemeric nucleic acid molecular structures; and (c) dendrimers.

The term "high molecular weight polymers" as used in connection with the present invention refers to linear or branched molecule consisting of several identical blocks or of several types of identical blocks with a molecular mass from 10³ Da to 10⁹ Da, more preferably 10⁴ Da to 10⁸ Da and even more preferred 10⁵ Da to 10⁷ Da.

Preferably, said high molecular weight polymers as used in connection with the present invention are linear polyacrylamides, dextranes, or polyethyleneglycols.

The term "concatemeric nucleic acid molecular structures" refers to products of rolling circle or branched rolling circle amplification [Nallur et.al., 2001].

The term "dendrimer" as used in connection with the present invention refers to a synthetic, three-dimensional macromolecule of fractal nature. A dendrimer is built up from a monomer, with new branches added in steps until a tree-like structure is created [Tomalia, 2005].

In a more preferred embodiment of the method of the present invention, said solid material is selected from the group consisting of (a) polyacrylamide granules; (b) latex particles or (c) silica particles.

It is preferred in the method of the present invention that polyacrylamide granules of 3 to 20% w/v, and more preferably of 5 to 10% w/v of crosslinked polyacrylamide are prepared. The size of said granules preferably is in a range of 1 to 500 micron, more preferably in the range of 10 to 400 micron, 20 to 300 micron or 30 to 200 micron be prepared by water-in-oil emulsion technique [Cretich et.al., 2003].

It is also preferred in the method of the present invention that the strands of the duplex nucleic acid molecules which will be covalently attached to polyacrylamide granules have a concentration of 0,1-100µM, more preferably of 0,2-10µM.

Also, the use of latex particles and silica particles for attaching nucleic acid molecules are known to the person skilled in the art. [Elaissari et. al. 2003]

In still another preferred embodiment of the method of the present invention, said attached strands are used as templates for primer extension before electrophoresis is performed.

The person skilled in the art knows how to carry out the primer extension procedure. (Sambrook et. al.).

It is more preferred that said primer extension is the Sanger arrested primer extension with fluorescently labelled ddNTPs.

In still another preferred embodiment of the method of the present invention said heating of the samples during or before electrophoresis is performed, is carried out at temperatures suitable for melting said duplex nucleic acid molecules.

It is more preferred that said temperature is higher than 45°C.

Preferably, said temperature is higher than 60°C, 65°C and most preferred higher than 70°C.

In still another preferred embodiment of the method of the present invention said method further comprises the steps of (a') and (c'): (a') performing the Sanger sequencing reaction on said strands which are attached to a macromolecular structure or solid material, and (c') assessing the sequence of said strands which are attached to a macromolecular structure or solid material, wherein step (a') is carried out before said loading of a sample containing nucleic acid molecules onto a porous matrix is carried out and wherein step (c') is carried out after performing electrophoresis in said porous matrix.

Furthermore, the present invention relates to a method of sequencing comprising the steps of (a) performing the Sanger sequencing reaction on said strands which are attached to a macromolecular structure or solid material; (b) loading a sample containing duplex nucleic acid molecules onto a porous matrix, wherein one strand of each duplex nucleic acid molecule is attached to a macromolecular structure or solid material; (c) heating the sample of step (b), thereby melting said duplex nucleic acid molecules; and, concomitantly or subsequently;(d) performing electrophoresis in said porous matrix; and (e) assessing the sequence of said strands which are attached to a macromolecular structure or solid material.

It is preferred in the method of the present invention that said nucleic acid strands attached to polyacrylamide granules are prepared by the extension of the oligonucleotide primers preliminary attached to the granules.

The primer extension may be performed by insertion of the polyacrylamide granules in the individual conventional PCR reactions, where said attached primers are complementary to the one strand of PCR product. Preferably, said PCR reactions have less amount of one primer then another in liquid phase as described previously [Dressman et al. 2003]. During PCR amplification the attached primers will be elongated on the template of said strand of PCR product.

Another possibility to perform extension of the primer attached to the polyacrylamide granules is to perform PCR amplification in emulsion of said polyacrylamide granules in oil with detergent [Dressman et al. 2003].. It is preferred that 1-10 volumes of oil (relative to a volume of polyacrylamide granules) with 2-7% of Span-80 and 0,2-1 % of Tween-80 be used. It is preferred that number of initial templates in the PCR reaction (individual DNA molecules or DNA concatemers or bacteria with cloned DNA) were less than number of polyacrylamide granules. Oil and detergents are used to isolate individual granules from each other [Tawfik and Griffiths, 1998]. PCR reaction in the individual granules are based on the amplification procedure described in [Mitra and Church, 1999]

After PCR amplification the polyacrylamide granules are washed from the components of PCR reaction. The strand, which is not attached to the granules, is removed by washing granules at denaturing conditions at high temperature or by the procedure described in Example 2.

Sanger extension with four fluorescently labeled ddNTPs of step (a) is performed for several different granules in the same tube [ref.: Sambrook, 2001]. Commercial kits (for example, from Applied Biosystems: BigDye® Terminator v3.1 Cycle Sequencing Kit) may be used for carring out this reaction according to the manufacturer's protocol but without cycling (perform only one sequencing cycle). Then unincorporated nucleotides and primers are washed out from granules in nondenaturating conditions.

Polyacrylamide granules are loaded onto the gel as described for example in Example 2. After electrophoresis fluorescent bands are detected by fluorescent detection optical system (e.g. fluorescent microscope). Analysis of relative mobility of products labeled by different fluorophores allows to determine the sequence of the part of the attached nucleic acid strands.

**The figures show:**
**Figure 1:** Sequences of DNA duplexes of Example 2: (a) 20nt duplex; (b) 98nt duplex.
**Figure 2:** Thermal stability (8°C, 15°C, 25°C, 35°C, 45°C, 55°C, 65°C, 75°C, 85°C) of 20nt DNA duplexes attached to polyacrylamide gel in the presence of different concentration of urea (0%, 12%, 22%, 32%, 42%).
**Figure 3:** Thermal stability (8°C, 15°C, 25°C, 35°C, 45°C, 55°C, 65°C, 75°C, 85°C) of 98nt DNA duplexes attached to polyacrylamide gel in the presence of different concentration of urea (0%, 12%, 22%, 32%, 42%).
**Figure 4:** Using of polyacrylamide granules for loading DNA samples on electrophoresis in denaturing conditions Lanes 1-5: electrophoresis from polyacrylamide granules; . Lanes 6-9: electrophoresis with normal loading (500fmol; 250fmol; 100fmol and 50fmol of each fragment per lane).

The examples illustrate the invention.

### MATERIAL AND METHODS

Sequences of oligonucleotides are presented in Table 1. Acrydite-modified oligonucleotides were prepared by Eurogentec (Belgium), the other oligonucleotides were prepared by TIB Molbiol (Germany). Terminal Transferase, Polynucleotide Kinase (PNK), Taq polymerase, exo(-) Klenow polymerase, Single Strand Binding Protein (SSBP) were obtained from New England BioLabs (USA). Hot-start Taq polymerase was from Applied Biosystems (USA). All chemicals were from Sigma (USA).

5'-end labeling of oligonucleotides was performed at 37°C for 1 h. 100pmol of oligonucleotide was incubated in 10µl of T4 PNK buffer (70mM Tris-HCl pH 7.6, 10mM MgCl₂, 5mM DTT) with 30µCi [γ-³²P]dATP and 5u of T4 PNK followed by heat inactivation of the enzyme at 85°C for 15 min.

3'-end labeling of oligonucleotides was performed by terminal transferase at 37°C for 30min. 500pmol of oligonucleotide were incubated in 10µl of 1x "NEB bufer 4" with 0.25mM CoCl₂, 0.1mM dATP, 30µCi [α-³²P]dATP and 5u of Terminal Transferase (New England Biolabs). Terminal Transferase was then heat inactivated at 85°C for 15min.

*PCR amplification in polyacrylamide gel layer (modification of the protocol described in [*Mitra and Church, 1999*])*.
To cast the polyacrylamide gels for PCR reaction, 200µl of the following PCR mix was prepared: 1xPCR buffer (*10mM Tris-HCl pH 8, 50mM KCl, 0.01mM EDTA, 0.05% Tween20*), 2.5mM MgCl₂, 0.2mM dNTPs, 0.2% BSA, 0.1% Tween 20, 1µM PCR primer #acr6 (5' acrydite-modified), 0.5µM PCR primer #a122, 10% acrylamide : bisacrylamide (199:1), 0.5% DATD (N,N'-Diallyltartardiamide), 0.17u/µl hot-start Taq polymerase. As a template, cloned PCR fragment #template was used at concentration 0.02zmol/µl. 2µl of 10% ammonium persulfate and 2µl of 10% TEMED were added to PCR mix. The mix was poured between silanised glass slide and silanised cover slip separated by 0.5mm thick plastic spacers. The mix was allowed to polymerise for 10min at room temperature. Then a secure seal hybridisation chamber (Grace Bio-labs) was placed on the slide covering the gel, and mineral oil was pipetted into the chamber. The slide was then placed in a PCR block with flat surface and PCR was performed (94° for 10min, 40 cycles of 94°C for 45sec, 61 °C for 45sec, 68°C for 45sec).

### Examples:

### Example 1:

Attachment of acrydite-modified oligonucleotides in the polyacrylamide gel via copolymerization.

Four oligonucleotides (unmodified oligonucleotides #a030 and #a077, and 5'-acrydite modified oligonucleotides #acr23 and #acr528) were 3'-labeled by terminal transferase. Then, these oligonucleotides were separately polymerized for 1 h at normal temperature in 0,5mm thick 5x5mm gel pieces of 10% acrylamide : bisacrylamide (199:1), 0.5% DATD (N,N'-Diallyltartardiamide), 0,05% Ammonium persulfate, 0,05% TEMED (protocol is based on the protocol described in [Rehman et al, 1999]).

Obtained pieces of gel, each containing one of the four oligonucleotides, were subjected to several washes in 1xTE buffer (100mM TrisCl; 1mM EDTA; pH 8):
"wash I" - 20°C, 30min;
"wash II" - 20°C, 30min;
"wash III" - 85°C, 30min;
"wash IV" - 85°C, 2h;
Each washing step was performed in 2ml tube with 1ml of 1xTE in Thermomixer (Eppendorf) with constant shaking (800rpm). After each washing step, the washing buffer was removed and the radioactivity of gel pieces was measured by hand-held radioactivity-meter. The results of radioactivity measurements (counts per second) are presented in Table 2.

This example demonstrates that acrydite-modified oligonucleotides may be efficiently attached to polyacrylamide gel by copolymerization. Table 2 shows, that unmodified oligonucleotides continuously diffuse from the gel pieces during washing, whereas about 50% of acrydite-modified oligonucleotide remain in the gel pieces after the stringent wash.

### Example 2:

Thermal stability of DNA duplexes attached to polyacrylamide gel in the presence of different concentration of urea.
98bp DNA duplex was prepared by PCR amplification in 0.5 mm thick polyacrylamide gel. After PCR, the gel pad was washed several times in 1 x TE buffer. The PCR products were denatured by incubating the gel in a formamide buffer (1xSSC, 70% formamide) at 70°C for 15min. Immediately following denaturing, the gel was electrophoresed to remove the non-attached strands in 0.5xTBE buffer with 42% urea.

For the first series of experiments 5'-labeled 20nt primer was hybridized to the single-stranded DNA in the gel. The gel was washed 2x in Washing Buffer (100mM Tris-HCl pH 7.5, 20mM EDTA, 500mM KCI). Then it was placed on a silanised glass slide and 200µl of Annealing Buffer (6xSSPE, 0.01% Triton X-100) with 0.5µM 5'-labeled primer #a122 were pippeted onto the gel and a secure seal hybridisation chamber (Grace Bio-labs) was placed on the slide covering the gel. The slide was placed in a PCR block with a flat surface and annealing was performed: (94°C for 2min, 56°C for 15min).

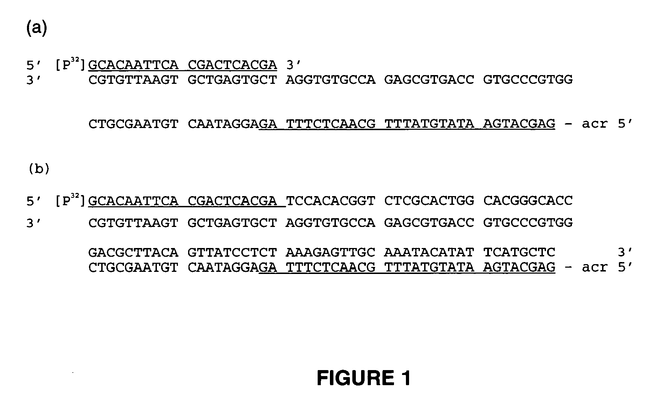

For the second series of experiments hybridized oligonucleotides were extended by Klenow exo(-) DNA polymerase (Fig.1). Piece of gel (~10x10mm) with annealed #a122 was equilibrated in 1x Klenow buffer (50mM Tris-HCl, pH 7.5, 5mM MgCl₂, 7.5mM DTT). 15µl of extension mix (1x Klenow buffer, 25µM dNTPs, 495ng of SSBP, 1u/µl Klenow (exo-) polymerase) were added to the to the gel. The extension was performed at 37°C for 15min.

Five peaces of each gel (with extended and not extended primer) with the volume of each peace about 5µl were washed 2x at 0°C in 1,5ml {1xTBE with different concentrations of urea: 0%, 12%, 22%, 32%, 42% } and placed in individual tubes. The peaces were cut in about 0,5mm³ cubes and 15µl of {1xTBE, correspondent concentration of urea} were added to each tube. Nine steps of elution were performed on the Eppendorf Thermomixer: shaking at 800rpm for 15 min at each temperature (8°C, 15°C, 25°C, 35°C, 45°C, 55°C, 65°C, 75°C, 85°C). After each elution step gel was pelleted by centrifugation and radioactivity in 5ul of supernatant was measured by hand-held radioactivity-meter. The supernatant was then returned to the tube and the next elution step was performed.

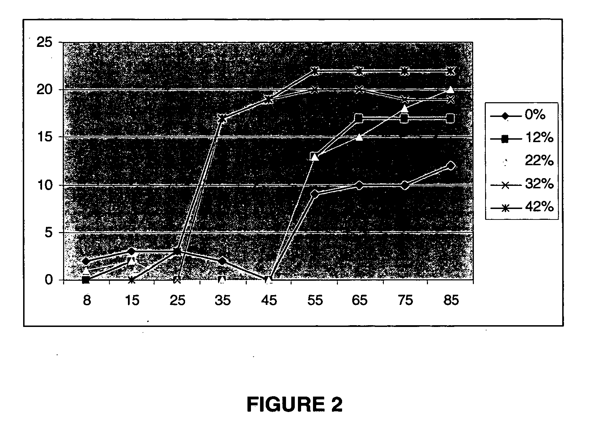

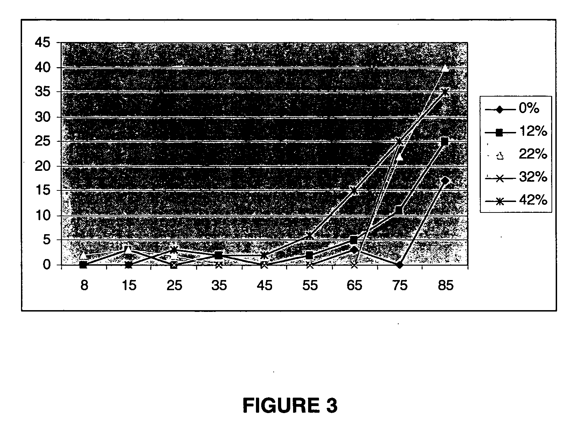

The results of radioactivity measurement (counts per second) are presented in tables 3 and 4 and in Figures 2 and 3.

This Example shows that DNA duplexes are stable at low temperatures (up to 25°C for 20nt hybrid and up to 45°C for 98nt hybrid) in spite of high concentration of urea.

### Example 3:

Use of polyacrylamide granules for loading DNA samples on electrophoresis in denaturing conditions.

30 polyacrylamide granules were prepared by polymerization of 0.5µl droplets of {10% acrylamide : bisacrylamide (199:1), 0.5% DATD (N,N'-Diallyltartardiamide), 0,05% Ammonium persulfate, and 100fmol of each PCR-fragment} under nitrogen-saturated oil with 0,4% TEMED. Four different PCR fragments (249bp, 225bp, 204bp, 52bp) with one unmodified and one acrydite-modified primer were copolymerized in granules.

#194acr - #237 52nt
5′
GACGTTGTAA AACGACGGCC AGTGAGCGCG CGTAATACGA CTCACTATAG GG 3′

#194acr - #221 204nt

5′
GACGTTGTAA AACGACGGCC AGTGAGCGCG CGTAATACGA CTCACTATAG
GGCGAATTGG GTACCGGGCC CCCCCTCGAG GTCGACGGTA TCGATAAGCT
TGATATCGAA TTCCTGCAGC CCGGGGGATC CACTAGTTCT AGAGCGGCCG
CCACCGCGGT GGAGCTCCAG CTTTTGTTCC CTTTAGTGAG GGTTAATTGC GCGC 3′

#194acr - #147 233nt
5′
GACGTTGTAA AACGACGGCC AGTGAGCGCG CGTAATACGA CTCACTATAG
GGCGAATTGG GTACCGGGCC CCCCCTCGAG GTCGACGGTA TCGATAAGCT
TGATATCGAA TTCCTGCAGC CCGGGGGATC CACTAGTTCT AGAGCGGCCG
CCACCGCGGT GGAGCTCCAG CTTTTGTTCC CTTTAGTGAG GGTTAATTGC
GCGCTTGGCG TAATCATGGT CATAGCTGTT TCC 3′

#194acr - #195 249nt
5′
GACGTTGTAA AACGACGGCC AGTGAGCGCG CGTAATACGA CTCACTATAG
GGCGAATTGG GTACCGGGCC CCCCCTCGAG GTCGACGGTA TCGATAAGCT
TGATATCGAA TTCCTGCAGC CCGGGGGATC CACTAGTTCT AGAGCGGCCG
CCACCGCGGT GGAGCTCCAG CTTTTGTTCC CTTTAGTGAG GGTTAATTGC
GCGCTTGGCG TAATCATGGT CATAGCTGTT TCCTGTGTGA AATTGTTAT 3′

After polymerization granules were washed 3 times at normal temperature in 1,5ml of 1xTBE and stored at 4°C. Five granules were washed 2x in 1,5ml {1xTBE, 42% urea} at normal temperature and placed between electrophoresis glasses. Then, electrophoresis gel {20% Acrylamide:Bis-Acrylamide (19:1), 1xTBE, 42% urea, 0,05% Ammonium persulfate, 0,05% TEMED} was poured between these glasses.

Four samples with the same four PCR fragments (500fmol; 250fmol; 100fmol and 50fmol of each fragment per lane) were loaded on the gel by the normal way. Immediately before electrophoresis 100ml of preheated (90°C) 1xTBE buffer were poured in the upper chamber to heat the gel to about 75°C. After electrophoresis the gel was stained by SYBR Green II.

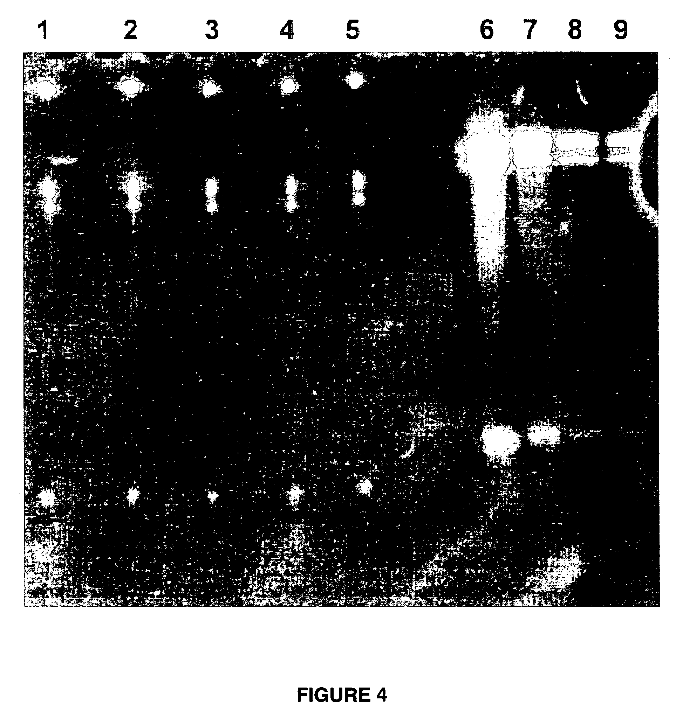

The picture of the gel (Fig. 4) shows, that the quality of the electrophoretic separation is the same for normal loading and loading DNA in granules

Example 3 shows that nucleic acids may be loaded on the gel in the form of doublestranded hybrids, where one strand will be analysed by electrophoresis and complementary strand serves to prevent diffusion of the sample before electrophoresis. The duplexes should be denatured by heating immediately before or during electrophoresis.

### References

Arshady R (1993) Micro-spheres for biomedical applications: preparation of reactive and. labeled micro-spheres. Biomaterials 14: 5-15
Burgess JK. Gene expression studies using microarrays. Clin Exp Pharmacol Physiol. 2001 Apr;28(4):321-8. Review.
Cretich M, Pirri G, Carrea G, Chiari M. Separation of proteins in a multicompartment electrolyzer with chambers defined by a bed of gel beads. Electrophoresis. 2003 Feb;24(4):577-81.
Dong S, Wang E, Hsie L, Cao Y, Chen X, Gingeras TR. Flexible use of high-density oligonucleotide arrays for single-nucleotide polymorphism discovery and validation. Genome Res. 2001 Aug;11(8):1418-24.
Dressman D, Yan H, Traverso G, Kinzler KW, Vogelstein B. Transforming single DNA molecules into fluorescent magnetic particles for detection and enumeration of genetic variations. Proc Natl Acad Sci U S A. 2003 Jul 22;100(15):8817-22. Epub 2003 Jul 11.
Elaissari, A.; Ganachaud, F.; Pichot, C. Biorelevant Latexes and Microgels for the Interaction with Nucleic Acids, Topics in current chemistry, 2003; VOL 227; 169-194
Fritzsche, W.; Taton, TA "Metal Nanoparticles as Labels for Heterogeneous, Chip-Based DNA. Detection." Nanotechnology 2003, 14, R63-R73
Hacia, J. G. Resequencing and mutational analysis using oligonucleotide microarrays Nature Genetics 21 (1 Suppl), 42-7, 1999
Hermanson G. Bioconjugate Techniques Elsevier Science; 1996; 785cTp.; ISBN: 0123423368
Huang SC, Swerdlow H, Caldwell KD. Binding of biotinylated DNA to streptavidin-coated polystyrene latex. Anal Biochem. 1994 Nov 1;222(2):441-9.
Mitra RD, Church GM .In situ localized amplification and contact replication of many individual DNA molecules. Nucleic Acids Res. 1999 Dec 15;27(24):e34.
Nallur G, Luo C, Fang L, Cooley S, Dave V, Lambert J, Kukanskis K, Kingsmore S, Lasken R, Schweitzer B. Signal amplification by rolling circle amplification on DNA microarrays. Nucleic Acids Res. 2001 Dec 1;29(23):E118.
Paegel BM, Blazej RG, Mathies RA. Microfluidic devices for DNA sequencing: sample preparation and electrophoretic analysis. Curr Opin Biotechnol. 2003 Feb;14(1):42-50. Review.
Paegel BM, Yeung SH, Mathies RA. Microchip bioprocessor for integrated nanovolume sample purification and DNA sequencing. Anal Chem. 2002 Oct 1;74(19):5092-8.
Rehman FN, Audeh M, Abrams ES, Hammond PW, Kenney M, Boles TC. Immobilization of acrylamide-modified oligonucleotides by co-polymerization. Nucleic Acids Res. 1999 Jan 15;27(2):649-55.
Sambrook J. and Russell, D.W. (2001) Molecular cloning: a laboratory manual. NY, Cold Spring Harbor Laboratory press.
Shendure J, Mitra RD, Varma C, Church GM. Advanced sequencing technologies: methods and goals. Nat Rev Genet. 2004 May;5(5):335-44.
Shendure J, Porreca GJ, Reppas NB, Lin X, McCutcheon JP, Rosenbaum AM, Wang MD, Zhang K, Mitra RD, Church GM. Accurate Multiplex Polony Sequencing of an Evolved Bacterial Genome. Science. 2005 Aug 4; epub
Tawfik DS, Griffiths AD. Man-made cell-like compartments for molecular evolution. Nat Biotechnol. 1998 Jul;16(7):652-6.
Tomalia D.A., The dendritic state. Materials Today, 2005 March: 34-46.

### Tables:

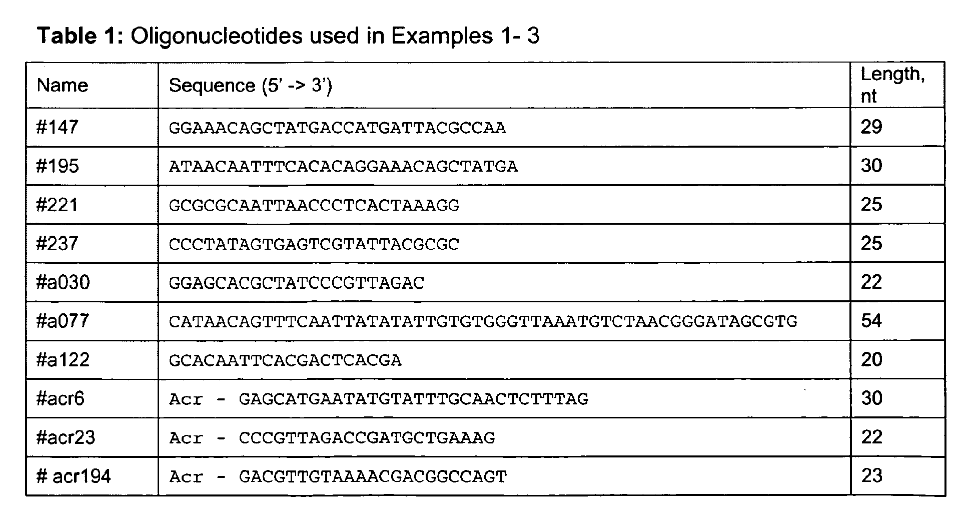

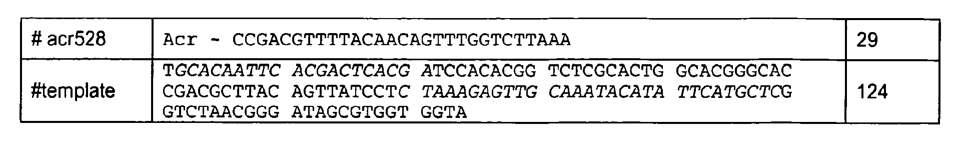

**Table 2: Stability of DNA oligonucleotides attached to polyacrylamide gel.**

| Oligonucleotide | Initial cps | cps after "wash I" | cps after "wash II" | cps after "wash III" | cps after "wash IV" |
|---|---|---|---|---|---|
| #a030 | 1000 | 380 | 200 | 70 | 40 |
| #a077 | 1100 | 380 | 130 | 45 | 25 |
| #acr23 | 1500 | 900 | 850 | 700 | 700 |
| #acr528 | 2000 | 1250 | 1200 | 1000 | 1000 |

**Table 3: Thermal stability of 20nt DNA duplexes attached to polyacrylamide gel in the presence of different concentration of urea.**

| Temperature | Concentration of urea | | | | |
|---|---|---|---|---|---|
| | 0% | 12% | 22% | 32% | 42% |
| 8°C | 2 | 2,5 | 1 | 1,5 | 1,5 |
| 15°C | 2 | 2 | 2 | 2,5 | 2,5 |
| 25°C | 2,5 | 1,5 | 1,5 | 1,5 | 3 |
| 35°C | 3 | 1,5 | 3,5 | 17 | 17 |
| 45°C | 4 | 12,5 | 12,5 | 19 | 19 |
| 55°C | 9 | 13 | 13 | 20 | 22 |
| 65°C | 10 | 17 | 15 | 20 | 22 |
| 75°C | 10 | 17 | 18 | 19 | 22 |
| 85°C | 12 | 17 | 20 | 19 | 22 |

**Table 4: Thermal stability of 98nt DNA duplexes attached to polyacrylamide gel in the presence of different concentration of urea.**

| Temperature | Concentration of urea | | | | |
|---|---|---|---|---|---|
| | 0% | 12% | 22% | 32% | 42% |
| 8°C | 3,5 | 1,5 | 2 | 1,5 | 1,5 |
| 15°C | 3,5 | 3 | 3 | 2,5 | 2,5 |
| 25°C | 2,5 | 2,5 | 2 | 1,5 | 3 |
| 35°C | 1,5 | 2 | 1,5 | 1,5 | 2 |
| 45°C | 2,5 | 1,5 | 1,5 | 1,5 | 2 |
| 55°C | 2,5 | 2 | 1,5 | 3,5 | 6 |
| 65°C | 3 | 5 | 6,5 | 6,5 | 15 |
| 75°C | 6,5 | 11 | 22 | 25 | 25 |
| 85°C | 17 | 25 | 40 | 35 | 35 |

## Claims

1. A method for performing an electrophoresis comprising the steps of
(a) loading a sample containing duplex nucleic acid molecules onto a porous matrix, wherein one strand of each duplex nucleic acid molecule is attached to a macromolecular structure or solid material;
(b) heating the sample of step (a), thereby melting said duplex nucleic acid molecules; and, concomitantly or subsequently,
(c) performing electrophoresis.

2. The method of claim 1, wherein said electrophoresis is carried out under denaturing conditions.

3. The method of claim 1 or 2, wherein said loading of the samples on the porous matrix is performed at a temperature wherein the duplex nucleic acid molecules are stable.

4. The method of claim 3, wherein said temperature is below 40°C.

5. The method of any one of claims 1 to 4, wherein said porous matrix is a gel.

6. The method of claim 5, wherein said gel is a polyacrylamide gel.

7. The method of any one of claims 1 to 6, wherein said porous matrix is a linear polymer.

8. The method of claim 7, wherein said linear polymer is a linear polyacrylamide.

9. The method of any one of claims 1 to 8, wherein said macromolecular structure is selected from the group consisting of
(a) high molecular weight polymers;
(b) concatemeric nucleic acid molecular structures; and
(c) dendrimers.

10. The method of any one of claims 1 to 8, wherein said solid material is selected from the group consisting of
(a) polyacrylamide granules;
(b) latex particles; and
(c) silica particles.

11. The method of any one of claims 1 to 10, wherein said strands which are attached to a macromolecular structure or solid material are used as templates for primer extension before electrophoresis is performed.

12. The method of claim 11, wherein said primer extension is the arrested primer extension with fluorescently labeled ddNTPs.

13. The method of any one of claims 1 to 12, wherein said heating of the samples during or before electrophoresis is performed, is carried out at temperatures suitable for melting said duplex nucleic acid molecules.

14. The method of claim 13, wherein said temperature is higher than 45°C.

15. A method of sequencing comprising the steps of
(a) performing the Sanger sequencing reaction on said strands which are attached to a macromolecular structure or solid material;
(b) loading a sample containing duplex nucleic acid molecules onto a porous matrix, wherein one strand of each duplex nucleic acid molecule is attached to a macromolecular structure or solid material;
(c) heating the sample of step (b), thereby melting said duplex nucleic acid molecules; and, concomitantly or subsequently;
(d) performing electrophoresis in said porous matrix; and
(e) assessing the sequence of said strands which are attached to a macromolecular structure or solid material.
